(19) Europäisches Patentamt
European Patent Office
Office européen des brevets

(11) **EP 3 964 128 A1**

(12) **EUROPÄISCHE PATENTANMELDUNG**

(43) Veröffentlichungstag:
**09.03.2022   Patentblatt 2022/10**

(21) Anmeldenummer: **21194550.6**

(22) Anmeldetag: **02.09.2021**

(51) Internationale Patentklassifikation (IPC):
**A61B 5/0507** (2021.01)   **A61B 5/00** (2006.01)
**A61B 5/11** (2006.01)   **G01S 13/00** (2006.01)

(52) Gemeinsame Patentklassifikation (CPC):
**A61B 5/0507; A61B 5/11; A61B 5/4519;
A61B 5/4528; A61B 5/4571; A61B 5/6804;
A61B 5/6823; A61B 5/6844; G01S 13/10;
G01S 13/32;** A61B 2560/0223; A61B 2562/046

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(30) Priorität: **04.09.2020   DE 102020123168**

(71) Anmelder: **Technische Universität Dresden
01069 Dresden (DE)**

(72) Erfinder:
 • **PLETTEMEIER, Dirk
01187 Dresden (DE)**
 • **STATZ, Christoph
01187 Dresden (DE)**
 • **ZHANG, Hui
01237 Dresden (DE)**
 • **LAABS, Martin
01097 Dresden (DE)**
 • **WANG, Qiong
01237 Dresden (DE)**
 • **HAMPE, Jochen
01277 Dresden (DE)**
 • **GÜNTHER, Klaus-Peter
01259 Dresden (DE)**
 • **GORONZY, Jens
01259 Dresden (DE)**

(74) Vertreter: **Viering, Jentschura & Partner mbB
Patent- und Rechtsanwälte
Am Brauhaus 8
01099 Dresden (DE)**

(54) **ERFASSUNGSVORRICHTUNG UND VERFAHREN ZUM BETREIBEN DERSELBEN**

(57) In verschiedenen Ausführungsformen weist eine Erfassungsvorrichtung (100) auf: einen verformbaren Träger (102) der einen vorbestimmten Bereich (110) zumindest teilweise umgeben kann, Erfassungseinheiten (104), die an dem Träger (102) befestigt sind und erste HF-Strahlung (HF) in Richtung eines Erfassungsbereiches (108) emittieren und zweite HF-Strahlung (HF) aus dem Erfassungsbereich (108) empfangen, wobei die empfangene zweite HF-Strahlung (HF) eine oder mehrere Eigenschaften des Erfassungsbereichs (108) repräsentiert und auf der emittierten ersten HF-Strahlung (HF) basiert. Eine Relativposition der Erfassungseinheiten kann aufgrund einer Verformung des Trägers (102) veränderbar sein. Eine Auswertevorrichtung (106) kann die Relativpositionen der Erfassungseinheiten basierend auf der empfangenen zweiten HF-Strahlung ermitteln. Eine Ausgabe wird basierend auf der ermittelten Relativposition bereitgestellt.

**FIG. 1A**

**Beschreibung**

Verschiedene Ausführungsbeispiele betreffen eine Erfassungsvorrichtung und ein Verfahren zum Betreiben derselben.

Derzeit herkömmliche routinemäßige medizinische Bildgebungstechniken basieren auf Röntgenstrahlen (beispielsweise Computertomographie (CT)), Ultraschall (US) oder Magnetresonanztomographie (MRT).

Bei der Bildgebung von Erkrankungen des Bewegungsapparates wird üblicherweise die Röntgenbildgebungstechnik (CT) zur Beurteilung der einseitigen Knochenpathologie und zur Diagnose eines Knochenbruchs und/oder einer Gelenkluxation verwendet. Ein medizinischer Röntgenscanner ist relativ gesehen kostengünstiger als ein MRT-Scanner, aber für die Bildgebung von Weichteilen schlecht und birgt die Gefahr ionisierender Strahlung, durch die organische Zellen geschädigt werden können, sodass es nicht lange bei Patienten, insbesondere bei Kindern und schwangeren Frauen, angewendet werden kann.

Im Unterschied zur Röntgenaufnahme (oder CT) wird die MRT in der muskoloskelletalen Diagnsotik vornehmlich zur Abbildung von nicht im Röntgen sichtbaren Gelenkstruktren wie Menisken oder Labren aber auch zur Beurteilung des Knorpels und periartikulärer Strukturen wie Sehnen oder Musekeln verwendet. Weiterführend kann im MRT auch eine hervorragende Beurteilung des Knochens durchgeführt werden, wobei diese vor allem für Tumorfälle oder schwierige mit Röntgen unzureichend darstellbare Fälle vorbehalten ist. Hochauflösende Bilder im MRT benötigten längere Unteruschungszeiten und sind deswegen in der standardisierten Bildegbung sehr kostenintensiv. Auch ist die MRT nicht für alle Patienten, da keine metallischen Objekte wie Herzschrittmacher etc. dem Magnetfeld ausgesetzt werden dürfen. Das bei MRT von den Magneten im System erzeugte Rauschen und die Enge Röhre in der sich der Patient während der Untersuchung befindet kann zudem dazu führen, dass sich einige Patienten während der MRT-Untersuchung klaustrophobisch fühlen.

Darüber hinaus sind MRT und CT —Systeme nicht und Röntgengeräte nur eingeschränkt mobil und erfordern eine kostenintensive Infrastruktur.

US ist zwar tragbarer und kostengünstiger als CT oder MRT, kann jedoch nur im Nahbereich hochauflösenden Bilder erfassen und der Anwendungsbereich der US-Bildgebung ist begrenzt. So ist es nicht möglich, Gewebe hinter Knochen und Luft abzubilden. Im Hinblick auf eine Untersuchung des Bewegungsapparates wird US verwendet, um den periartikulären weichen Bewegungsapparat zu bewerten und einen Gelenkerguss oder eine Synovialverdickung des Knochens festzustellen.

Die herkömmlichen Bildgebungstechniken und Modalitäten für Erkrankungen, beispielsweise Erkrankungen des Bewegungsapparates, sind statisch und berücksichtigen keine funktionellen Aspekte. Eine direkte und nicht simulierte Bewertung der Bewegung und Funktion, beispielsweise eines Knochengelenks, ist mit den herkömmlichen Bildgebungstechnik bisher nicht möglich. Eine direkte Bewertung der Bewegung und Funktion eines Knochengelenks ist damit nicht möglich.

Es ist somit eine Aufgabe eine Erfassungsvorrichtung bereitzustellen, die ein oder mehrere der zuvor genannten Probleme reduziert oder behebt, sowie ein Verfahren zum Betreiben derselben.

In einem Aspekt wird eine Erfassungsvorrichtung bereitgestellt, aufweisend einen verformbaren Träger, derart eingerichtet, dass ein vorbestimmter Bereich zumindest teilweise von dem Träger umgebbar ist; mindestens eine erste Erfassungseinheit und eine zweite Erfassungseinheit, die an dem Träger befestigt sind und jeweils eingerichtet sind, HF-Strahlung in Richtung eines Erfassungsbereiches zu emittieren und HF-Strahlung aus dem Erfassungsbereich zu empfangen, wobei die empfangene HF-Strahlung eine oder mehrere Eigenschaften des Erfassungsbereichs repräsentiert und auf der emittieren HF-Strahlung basiert; wobei der Träger derart verformbar eingerichtet ist, dass eine Relativposition der ersten Erfassungseinheit zu der zweiten Erfassungseinheit aufgrund einer Verformung des Trägers veränderbar ist; und eine Auswertevorrichtung, die eingerichtet ist, die Relativposition der ersten und zweiten Erfassungseinheit zu ermitteln und eingerichtet ist, eine Ausgabe bereitzustellen basierend auf der ermittelten Relativposition der ersten und zweiten Erfassungseinheit und der mittels der ersten Erfassungseinheit und der zweiten Erfassungseinheit empfangenen HF-Strahlung.

Der verformbare Träger kann plastisch oder elastisch verformbar eingerichtet sein. Der Träger kann ein Gewebe oder einen Formkörper aufweisen oder daraus gebildet sein. Der Träger kann eine planare aufweisen, beispielsweise in Form eines Pflasters oder Verbands. Alternativ kann der Träger eine Schlauch-, Schlaufen- oder Hüllen-artige Form aufweisen, beispielsweise als ein Kleidungsstück, beispielsweise eine Unterhose, eine Socke, ein Handschuh etc..

In verschiedenen Ausführungsbeispielen kann der vorbestimmte Bereich der Erfassungsbereich sein, so dass der verformbare Träger den Erfassungsbereich umgibt. Die HF-Strahlung ist anschaulich somit in Richtung des vorbestimmten Bereiches bzw. nach innen gerichtet. Beispielsweise kann der verformbare Träger zur Untersuchung eines Gelenks eines Patienten als ein entsprechendes Kleidungsstück eingerichtet sein, beispielsweise als eine Hose oder Unterhose zur Untersuchung eines oder beider Hüftgelenke. Für andere Gelenke kann der verformbare Träger eine entsprechend andere Form aufweisen, beispielsweise die Form einer Socke für die Untersuchung eines Knöchels. Die Erfassungsvorrichtung kann somit die Form einer Unterhose, eines Hemds, einer Gesichtsmaske, einer Jacke, eines Schuhs, einer Socke, eines Handschuhs oder eines anderen Kleidungsstücks oder Accessoires haben, beispielsweise die Form eines Schals oder eines Ringes. Der verformbare Träger kann jedoch auch eine unspezifische Form aufweisen, beispielsweise in Form eines wickelbaren Verbands oder eines Pflasters, so dass unterschiedliche anatomische Bereiche umgeben bzw. erfasst werden können. Weiterhin wird dadurch ermöglicht, dass anatomisch sehr unterschiedliche Patienten untersucht werden können, beispielsweise kleine und große Patienten, Patienten mit starkem Übergewicht mit entsprechend unterschiedlich großen anatomischen Bereichen. Weiterhin können im veterinärmedizinischen Bereich damit unterschiedliche Tierarten mit einer einzigen Erfassungsvorrichtung untersucht werden, beispielsweise Pferde und Hunde.

In verschiedenen Ausführungsformen kann der vorbestimmte Bereich, der von dem verformbaren Träger umgeben wird, von dem Erfassungsbereich verschieden sein. Die HF-Strahlung ist anschaulich somit von dem vorbestimmten Bereich weg gerichtet bzw. nach außen zu dem Erfassungsbereich gerichtet. Anschaulich kann die Erfassungsvorrichtung in diesem Fall als ein Ergänzungsmodul bzw. ein Adapter, beispielsweise für ein bereits vorhandenes Diagnose- oder Behandlungsgerät, eingerichtet sein. Beispielsweise kann der verformbare Träger als ein Handschuh, Ring oder Fingerling eingerichtet sein (Handscanner), der von einem medizinischen Personal getragen wird, um einen Patienten zu untersuchen. Der Patient kann in diesem Fall untersucht werden, indem das medizinische Personal mit dem Handscanner den Körper des Patienten berührt oder darüber anordnet. Als weiteres Beispiel kann der verformbare Träger auf ein Operationsbesteck aufgepflanzt werden und nach der Benutzung von dem Operationsbesteck separiert desinfiziert werden oder entsorgt werden (Einmalprodukt).

Anschaulich können sich die Erfassungseinheiten mit einer Verformung des verformbaren Trägers mitbewegen. Mit anderen Worten: wenn der Träger im Betrieb oder zur Inbetriebnahme verformt wird, beispielsweise durch eine Bewegung eines zu untersuchenden Gelenks Körperbereichs oder durch Aufpflanzen (Ergänzungsmodul), verändert sich der relative Abstand bzw. die relative Position der Erfassungseinheiten zueinander, beispielsweise dynamisch. Dadurch kann die Auswertung der zweiten HF-Strahlung, beispielsweise die Bildgebung, zeitabhängig bzw. Erfassungseinheiten-positionsabhängig werden. Diese Abhängigkeit wird von der Auswertevorrichtung in der Auswertung der empfangenen zweiten HF-Strahlung berücksichtigt, wie unten noch ausführlicher beschrieben wird. Dies ermöglicht beispielsweise eine dynamische Bildgebung. Alternativ oder zusätzlich wird eine Erfassungsvorrichtung für den Einsatz im Feld (beispielsweise nicht-stationär) ermöglicht.

Eine Erfassungseinheit kann anschaulich ein Sensor oder eine Gruppe von Sensoren sein. Eine Erfassungseinheit kann beispielsweise eine oder mehrere Antennen aufweisen, beispielsweise eingerichtet in Funktion einer Dipolantenne oder einer Antennenspule; ein Antennen-Array mit einer Vielzahl von Antennen, beispielsweise in Funktion einer Gruppenantennen oder einer Phased-Array-Antenne oder als eine Vielzahl funktional separierter Antennen. Die HF-Strahlung (hochfrequente Strahlung) kann eine elektromagnetische Strahlung in einem Bereich von 100 MHz bis 10 THz sein. Die Erfassungseinheiten bzw. deren Antennen können eingerichtet sein, eine HF-Strahlung in diesem Frequenzbereich zu senden bzw. zu emittieren und zu empfangen bzw. detektieren. Empfangen bzw. Detektieren bedeutet in diesem Sinne, dass die zweite HF-Strahlung hinsichtlich, Frequenz, Einfallswinkel, Polarisation etc. von der Erfassungseinheit zumindest teilweise aufgenommen werden kann und in ein Datensignal umgewandelt werden kann.

Die erste Erfassungseinheit kann zweite HF-Strahlung empfangen, die zuvor zumindest teilweise von der ersten und/oder zweiten Erfassungseinheit als erste HF-Strahlung emittiert wurde. Dementsprechend kann eine Erfassungseinheit zweite HF-Strahlung(en) empfangen, die auf erste HF-Strahlung(en) zurückführbar ist, die von einer oder mehreren Erfassungseinheiten gleichzeitig, nacheinander oder in einer Reihenfolge emittiert wurde(n). Die Erfassungseinheiten können zum Emittieren und Empfangen gleicher oder unterschiedlicher HF-Strahlungen eingerichtet sein. Beispielsweise kann eine erste Erfassungseinheit eingerichtet sein, eine HF-Strahlung eines ersten Frequenzbereiches zu emittieren und eine zweite Erfassungseinheit kann eingerichtet sein, eine HF-Strahlung eines zweiten Frequenzbereiches, der von dem ersten Frequenzbereich unterscheidbar beabstandet ist, zu emittieren. Die erste und zweite Erfassungseinheiten können die HF-Strahlungen gleichzeitig oder nacheinander emittieren. Eine dritte Erfassungseinheit kann unteranderem eingerichtet sein, die HF-Strahlung des ersten und zweiten Frequenzbereiches zu empfangen. Mittels der unterschiedlichen ersten und zweiten Frequenzbereiche kann die dritte Erfassungseinheit empfangene zweite HF-Strahlung der ersten bzw. zweiten Erfassungseinheit zuordnen und entsprechend auswerten.

Die Erfassungseinheiten können anwendungsspezifisch reversibel, beispielsweise abnehmbar, oder irreversibel, beispielsweise in dem Träger eingebettet oder integriert, an dem Träger befestigt sein.

Die Ausgabe kann anwendungsspezifisch eingerichtet sein. Beispielsweise kann die Ausgabe als ein einfaches Hinweissignal, das ein vordefinierte Bedingung erfüllt ist, eingerichtet sein. Ein Hinweissignal ist beispielsweise ein akustisches Signal, beispielsweise ein Signalton, oder ein optisches Signal, beispielsweise eine Signalleuchte. Ein Hinweissignal als Ausgabe kann beispielsweise in einem Screening-Verfahren angewendet werden, wenn lediglich ein qualitatives Untersuchungsergebnis erforderlich ist oder möglich ist, beispielsweise aus Datenschutzrechtlichen Gründen oder wenn eine schnelle Voruntersuchung durchgeführt wird. Beispielsweise kann das Vorhandensein eines Fremdkörpers oder eines vorbestimmten Erkrankungssymptoms qualitativ durch ein Hinweissignal angezeigt werden. Im Nachgang kann eine ausführlichere Untersuchung am Patienten vorgenommen werden.

Die Ausgabe kann jedoch auch ein Datensignal, eine Bilddatei, eine Videodatei bzw. eine Bildfolge, ein Live-Stream, eine Vektorgrafik, eine Datenmatrix oder ähnliches sein und eine Vielzahl von Datenpunkten aufweisen.

In einem weiteren Aspekt ist ein Verfahren zum Betreiben einer zuvor beschriebenen Erfassungsvorrichtung bereitgestellt. Das Verfahren kann aufweisen: Ermitteln der Relativposition zwischen der ersten Erfassungseinheit und der zweiten Erfassungseinheit; Emittieren erster HF-Strahlung in den Erfassungsbereich mittels der ersten Erfassungseinheit und/oder der zweiten Erfassungseinheit; Erfassen mittels der ersten Erfassungseinheit und/oder der zweiten Erfassungseinheit zweiter HF-Strahlung; Bereitstellen mittels der Auswertevorrichtung einer Ausgabe für die erste Erfassungseinheit und/oder die zweite Erfassungseinheit basierend auf der Relativposition und der/den erfassten zweiten HF-Strahlung(en) .

Dies ermöglicht eine Untersuchung bzw. Diagnose eines Patienten, während sich der Erfassungsbereich, beispielsweise durch eine Bewegung des Patienten, zeitlich verändern kann. Beispielsweise wird eine direkte und dreidimensionale Visualisierung der Knochengelenkbewegung in Echtzeit ermöglicht. Dies ermöglicht neue Erkenntnisse über Gelenkpathologien und personalisierte Behandlungspläne, beispielsweise auf strahlungsfreie und entfernte (remote) Weise.

Durch Variation der Frequenz der zu emittierenden HF-Strahlung kann die Eindringtiefe in das Gewebe und somit die Diagnosetiefe eingestellt werden, beispielsweise die Oberfläche eines Patienten und/oder tieferliegendes Gewebe abgetastet werden.

Ausführungsbeispiele sind in den Figuren dargestellt und werden im Folgenden näher erläutert.

Es zeigen

FIG.1A und FIG.1B      schematisch eine Erfassungsvorrichtung gemäß verschiedenen Ausführungsformen;

FIG.2      ein Ablaufdiagram eines Verfahrens zum Betreiben einer Erfassungsvorrichtung gemäß verschiedenen Ausführungsformen;

FIG.3      ein Anwendungsbeispiel einer Erfassungsvorrichtung und eines Verfahrens zum Betreiben einer Erfassungsvorrichtung;

FIG.4A-E      ein Anwendungsbeispiel einer Erfassungsvorrichtung und eines Verfahrens zum Betreiben einer Erfassungsvorrichtung;

FIG.5      ein Diagramm einer Erfassungsvorrichtung;

FIG.6      ein Timing-Diagramm eines Verfahrens zum Betreiben einer Erfassungsvorrichtung; und

FIG.7      ein Diagramm eines Verfahrens zum Betreiben einer Erfassungsvorrichtung.

In der folgenden ausführlichen Beschreibung wird auf die beigefügten Zeichnungen Bezug genommen, die Teil der Beschreibung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende Beschreibung ist deshalb nicht in einschränkendem Sinne aufzufassen, und der Schutzumfang der vorliegenden Erfindung wird durch die angefügten Ansprüche definiert.

Anschaulich wird eine Erfassungsvorrichtung 100 bereitgestellt, die einen verformbaren Träger 102 aufweist, der einen vorbestimmten Bereich 110 zumindest teilweise umgeben kann, Erfassungseinheiten 104 sind an dem Träger 102 befestigt und emittieren erste HF-Strahlung in Richtung eines Erfassungsbereiches 108, und empfangen zweite HF-Strahlung aus dem Erfassungsbereich 108. Die empfangene zweite HF-Strahlung repräsentiert eine oder mehrere Eigenschaften des Erfassungsbereichs und basiert auf der emittierten ersten HF-Strahlung. Eine Relativposition der Erfassungseinheiten kann aufgrund einer Verformung des Trägers 102 veränderbar sein. Eine Auswertevorrichtung 106 kann die Relativpositionen der Erfassungseinheiten basierend auf der empfangenen zweiten HF-Strahlung ermitteln. Eine Ausgabe wird basierend auf der ermittelten Relativposition bereitgestellt.

Diese ermöglicht eine Ermittlung der Relativposition der Erfassungseinheiten 140 und eine Auswertung der Informationen aus dem Erfassungsbereich auf einfache Weise.

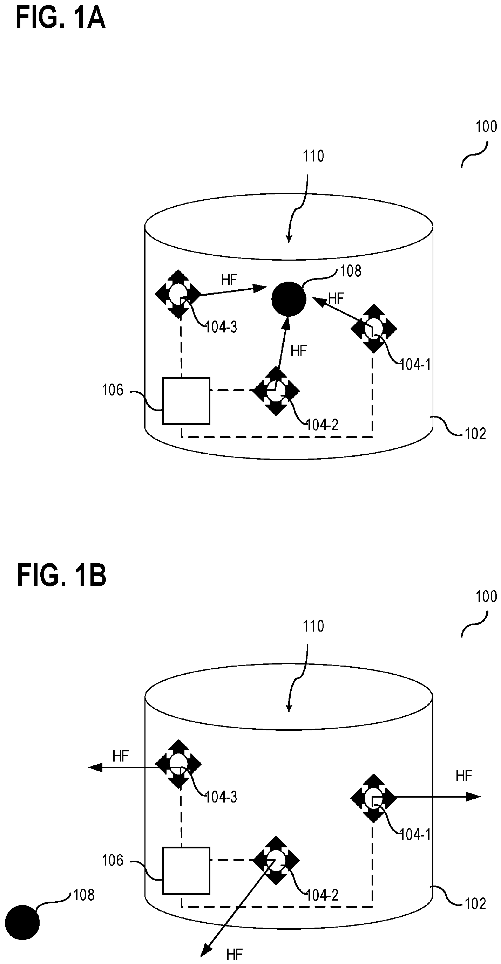

**FIG.1A** und **FIG.1B** veranschaulichen schematisch Erfassungsvorrichtungen 100 gemäß verschiedenen Ausführungsformen. Die Erfassungsvorrichtung 100 weist einen verformbaren Träger 102 auf. Der verformbare Träger 102 ist eingerichtet, dass ein vorbestimmter Bereich 110 zumindest teilweise von dem Träger 102 umgebbar ist. Der Träger 102 kann dabei eine planare Form aufweisen, beispielsweise in Form eines Pflasters, oder eine Hülsen-artige Form aufweisen, beispielsweise in Form eines Hohlzylinders.

In dem in FIG.1A veranschaulichten Ausführungsbeispiel ist ein Erfassungsbereich 108 bzw. ein zu erfassendes Objekt 108 in dem vorbestimmten Bereich 110 angeordnet (in FIG.1A mittels der bezogen auf den vorbestimmten Bereich 110 nach innen auf Objekt 108 gerichteten Pfeile veranschaulicht).

In dem in FIG.1B veranschaulichten Ausführungsbeispiel ist ein Erfassungsbereich 108 bzw. ein zu erfassendes Objekt 108 außerhalb des vorbestimmten Bereichs 110 angeordnet (in FIG.1B mittels der bezogen auf den vorbestimmten Bereich 110 nach außen auf Objekt 108 gerichteten Pfeile veranschaulicht). In diesem Fall kann die Erfassungsvorrichtung beispielsweise als Überzug, beispielsweise in Form eines Handschuhs, eines Rings oder eines Fingerlings als Handscanner, eingerichtet sein und verwendet werden. Anschaulich sind die Erfassungseinheiten 104 auf einem verformbaren Träger 102 befestigt. Der verformbare Träger 102 wird um einen vorbestimmten Bereich 110 gefügt. Der Träger 102 (und somit die Relativpositionen der Erfassungseinheiten 104 auf dem Träger 102) werden verformt. Als Beispiel, der Träger 102 hat die Form eines Handschuhs und der vorbestimmte Bereich 110 ist die Hand eines medizinischen Personals. Der vorbestimmte Bereich 110 wird durch die Erfassungsvorrichtung 100 zum "Diagnosegerät", um einen Patienten zu untersuchen und ist nicht der zu untersuchende Bereich (Erfassungsbereich) 108 des Patienten.

Mit anderen Worten: der vorbestimmte Bereich 110 kann zumindest teilweise den Erfassungsbereich 108 umfassen (FIG.1A). Alternativ oder zusätzlich ist der vorbestimmte Bereich 110 zumindest teilweise durch den Träger 102 von dem Erfassungsbereich 108 separiert (FIG.1B).

Der Erfassungsbereich 108 kann einen zeitlich veränderlichen Raum umfassen. Die zeitliche Veränderung kann ein sich veränderndes Volumen des Erfassungsbereiches 108 (beispielsweise durch eine Positionsänderung des Trägers 102 zum zu erfassenden Objekt), eine sich verändernde Zusammensetzung des Erfassungsbereiches 108 (beispielsweise durch eine Änderung des Gehalts von Körperflüssigkeiten, beispielsweise Dehydrierung oder innere Blutungen) und/oder eine sich verändernde Ausrichtung des Erfassungsbereiches relativ zu den Erfassungseinheiten 104, beispielsweise eine Gelenkbewegung, sein.

Die Erfassungsvorrichtung 100 ermöglicht eine Untersuchung bzw. Diagnose eines Patienten, während sich ein Erfassungsbereich 108, beispielsweise ein anatomischer Abschnitt des Patienten durch eine Bewegung des Patienten, zeitlich verändern kann. Beispielsweise wird eine direkte und dreidimensionale Visualisierung der Knochengelenkbewegung in Echtzeit ermöglicht. Dies ermöglicht neue Erkenntnisse über Gelenkpathologien und personalisierte Behandlungspläne.

Die Erfassungsvorrichtung 100 weist dazu eine Vielzahl von Erfassungseinheiten 104, beispielsweise mindestens eine erste Erfassungseinheit 104-1 und eine zweite Erfassungseinheit 104-2 und (optional) eine dritte Erfassungseinheit 104-3 (oder weitere Erfassungseinheiten 104-N mit N einer ganzen Zahl), auf. Allgemein können zwei oder mehr Erfassungseinheiten 104 vorgesehen sein.

Eine Erfassungseinheit 104 kann anschaulich ein Sensor oder eine Gruppe von Sensoren sein. Eine Erfassungseinheit 104 kann beispielsweise eine oder mehrere Antennen aufweisen, beispielsweise eingerichtet in Funktion einer Dipolantenne oder einer Antennenspule; ein Antennen-Array mit einer Vielzahl von Antennen, beispielsweise in Funktion einer Gruppenantennen oder einer Phased-Array-Antenne oder als eine Vielzahl funktional separierter Antennen. Die erste Erfassungseinheit 104-1 und die zweite Erfassungseinheit 104-2 können beispielsweise jeweils mindestens einen Radarsensor aufweisen. Jede der Erfassungseinheiten 104-1, 104-2, 104-3 kann mindestens eine Antenne aufweisen, die zum Emittieren der ersten HF-Strahlung HF und/oder Empfangen der zweiten HF-Strahlung HF eingerichtet ist. Jede der Erfassungseinheiten 104-1, 104-2, 104-3 kann ein Antennen-Array mit einer Vielzahl von Antennen aufweisen, die jeweils oder zusammen zum Emittieren der ersten HF-Strahlung HF und/oder Empfangen der zweiten HF-Strahlung HF eingerichtet sind.

Die Erfassungseinheiten 104 (beispielsweise 104-1, 104-2, 104-3) sind jeweils eingerichtet, erste HF-Strahlung HF in Richtung eines Erfassungsbereiches 108 zu emittieren und zweite HF-Strahlung HF aus dem Erfassungsbereich 108 zu empfangen. Die empfangene zweite HF-Strahlung HF repräsentiert eine oder mehrere Eigenschaften des Erfassungsbereichs 108. Die empfangene zweite HF-Strahlung HF kann alternativ oder zusätzlich auf der emittierten ersten HF-Strahlung HF, die von einer der Erfassungseinheiten 104 zuvor emittiert wurde, basieren. Anschaulich können die erste Erfassungseinheit 104-1, die zweite Erfassungseinheit 104-2 und die dritte Erfassungseinheit 104-3 eine Ebene aufspannen und die Ebene kann in einigen Ausführungsbeispielen (FIG.1A) zumindest teilweise in dem Erfassungsbereich 108 angeordnet sein.

Die Erfassungseinheit 104 kann zur Erfassung der HF-Signale beispielsweise eines aufweisen aus: einem fotoleitenden Antennendetektor, einer elektrooptischen Abtastung und optischen Entzerrung, einem nichtlinearer Misch- und Differenzfrequenzdetektor, einem Glimmentladungsdetektor, einem Oberflächenplasmonendetektor, einem Golay-Detektor, einem Bolometer, einem thermoelektrischen Detektor, einem pyroelektrischen Detektor, einem Barrieredetektor, oder einem elektronengebundener Zustandsübergang-Detektor (Quantentopf-Detektor).

Die erste HF-Strahlung kann von dem zu untersuchenden Objekt in dem Erfassungsbereich 108 zumindest teilweise reflektiert, gestreut, transmittiert und/oder geleitet (beispielsweise als Oberflächenwelle) und re-emittiert werden. Die so erzeugte, zu empfangende zweite HF-Strahlung repräsentiert beispielsweise Struktur des Gewebes bzw. Körpers im Erfassungsbereich 108, beispielsweise die Muskelstruktur, -form, -position und deren elektromagnetische Eigenschaften.

Die von der ersten Erfassungseinheit emittierte erste HF-Strahlung kann von der ersten Erfassungseinheit 104-1 und/oder einer oder mehreren der anderen Erfassungseinheiten 104-2, 104-3 als zweite HF-Strahlung empfangen werden. Mit anderen Worten: die erste Erfassungseinheit 104 kann zweite HF-Strahlung empfangen, die zuvor zumindest teilweise von der ersten und/oder zweiten Erfassungseinheit 104 als erste HF-Strahlung emittiert wurde. Dementsprechend kann eine Erfassungseinheit 104 zweite HF-Strahlung(en) empfangen, die auf erste HF-Strahlung(en) zurückführbar ist, die von einer oder mehreren Erfassungseinheiten 104 gleichzeitig, nacheinander oder in einer Reihenfolge emittiert wurde(n). Die HF-Strahlung (hochfrequente Strahlung) kann eine elektromagnetische Strahlung in einem Bereich von 100 MHz bis 10 THz sein. Die Erfassungseinheiten 104 bzw. deren Antennen können eingerichtet sein, eine HF-Strahlung in diesem Frequenzbereich zu senden bzw. zu emittieren und zu empfangen bzw. detektieren. Empfangen bzw. detektieren bedeutet in diesem Sinne, dass die zweite HF-Strahlung hinsichtlich, Frequenz, Einfallswinkel, Polarisation etc. von der Erfassungseinheit 104 zumindest teilweise aufgenommen werden kann und in ein Datensignal umgewandelt werden kann. Die erste HF-Strahlung HF und die zweite HF-Strahlung HF können die gleiche Frequenz aufweisen.

Die Erfassungseinheiten 104 können anwendungsspezifisch reversibel, beispielsweise abnehmbar, oder irreversibel, beispielsweise in dem Träger 102 eingebettet oder integriert, an dem Träger 102 befestigt sein.

Der Träger 102 kann derart verformbar eingerichtet sein, dass eine Relativposition der ersten Erfassungseinheit 104-1 zu der zweiten Erfassungseinheit 104-2 aufgrund einer Verformung des Trägers 102 veränderbar ist (in FIG.1A und FIG.1B mittels der dickeren Pfeile veranschaulicht). Der Träger 102 ist beispielsweise aus einem elastischen oder plastischen Material gebildet, beispielsweise einem Gewebe aus einem Stoff oder Kunststoff oder aus einem elastischem oder plastischem Formkörper. Anschaulich können sich die Erfassungseinheiten 104 mit einer Verformung des verformbaren Trägers 102 mitbewegen. Mit anderen Worten: wenn der Träger 102 im Betrieb oder zur Inbetriebnahme verformt wird, beispielsweise durch eine Bewegung eines zu untersuchenden Körperbereichs oder durch Aufpflanzen (Ergänzungsmodul), verändert sich der relative Abstand bzw. die relative Position der Erfassungseinheiten 104 zueinander, beispielsweise dynamisch. Dadurch kann die Auswertung der zweiten HF-Strahlung, beispielsweise die Bildgebung, zeitabhängig bzw. Erfassungseinheiten-positionsabhängig werden. Diese Abhängigkeit wird von der Auswertevorrichtung 106 in der Auswertung der empfangenen zweiten HF-Strahlung berücksichtigt. Dies ermöglicht beispielsweise eine dynamische Bildgebung.

In verschiedenen Ausführungsbeispielen kann der Träger 102 eine Befestigungsstruktur aufweisen, beispielsweise einen Klettverschluss, einen Klebstofffilm, ein Gummiband oder einen anderen Verschlussmechanismus, die derart eingerichtet ist, dass sich der Träger 102 verformt, wenn sich der Erfassungsbereich 108 verformt.

Die Erfassungsvorrichtung 100 weist eine Auswertevorrichtung 106 auf, die eingerichtet ist, die Relativposition der Erfassungseinheiten 104 zueinander, beispielsweise der ersten und zweiten Erfassungseinheit 104-1, 104-2 zueinander, zu ermitteln. Die Auswertevorrichtung 106 ist ferner eingerichtet, eine Ausgabe bereitzustellen. Die Ausgabe basiert auf der ermittelten Relativposition, beispielsweise der ersten und zweiten Erfassungseinheit 104-1, 104-2 zueinander, und der mittels der ersten Erfassungseinheit 104-1 und der zweiten Erfassungseinheit 104-2 empfangenen HF-Strahlung HF.

Die Auswertung der empfangenen zweiten HF-Strahlung(en) in der Auswertevorrichtung 106 basiert auf einer (beispielsweise approximierten) Berechnung eines Integrals der erfassten Daten. Die erfassten Daten können sein:

$$F(M, V, t \ oder \ f)$$

wobei M die Messpunkte, V das Volumen, t die Zeit und f die Frequenz der HF-Strahlung ist. Die Intensität I in einem Messpunkt kann ermittelt werden aus:

$$\sum_{M} \int_{V} \int_{t \ bzw. f} F(M, V, t \ bzw. f) dV, dt \ bzw. df$$

Die Erfassungseinheiten 104 können eingerichtet sein, die Intensität, Laufzeit und/oder Phasenverschiebung der empfangenen zweiten HF-Strahlung beispielsweise bezogen auf ein Referenzsignal, beispielsweise die erste HF-Strahlung, zu erfassen. Die Auswertung der erfassten Daten kann auf einem Modell basieren, dass iterative verfeinert werden kann.

Die Ausgabe kann anwendungsspezifisch eingerichtet sein. Beispielsweise kann die Ausgabe als ein einfaches Hinweissignal, das ein vordefinierte Bedingung erfüllt ist, eingerichtet sein. Ein Hinweissignal ist beispielsweise ein akustisches Signal, beispielsweise ein Signalton, oder ein optisches Signal, beispielsweise eine Signalleuchte. Ein Hinweissignal als Ausgabe kann beispielsweise in einem Screening-Verfahren angewendet werden, wenn lediglich ein qualitatives Untersuchungsergebnis erforderlich ist oder möglich ist, beispielsweise aus Datenschutzrechtlichen Gründen oder wenn eine schnelle Voruntersuchung durchgeführt wird. Die Ausgabe kann jedoch auch ein Datensignal, eine Bilddatei, eine Videodatei bzw. eine Bildfolge, ein Live-Stream, eine Vektorgrafik, eine Datenmatrix oder ähnliches sein und eine Vielzahl von Datenpunkten aufweisen.

Die Auswertevorrichtung 106 kann eingerichtet sein, die Relativposition der ersten und zweiten Erfassungseinheit 104-1, 104-2 vor der Emission der ersten HF-Strahlung HF zu ermitteln. Alternativ oder zusätzlich ist die Auswertevorrichtung 106 eingerichtet, die Relativposition der ersten und zweiten Erfassungseinheit 104-1, 104-2 basierend auf der empfangenen zweiten Strahlung zu ermitteln.

Die Auswertevorrichtung 106 kann zumindest teilweise auf dem Träger 102 integriert sein. Alternativ kann die Auswertevorrichtung 106 Träger-extern vorgesehen sein, beispielsweise für den Fall, dass der Träger 102 mit Erfassungseinheiten 104 als Einmalprodukt eingerichtet ist.

Die Auswertevorrichtung 106 kann eine Radar-Bildgebung umfassen, beispielsweise eine Vektor-Radar-Bildgebung.

Anzeigevorrichtung aufweisen, die eingerichtet ist, die Ausgabe anzuzeigen.

Die Erfassungsvorrichtung 100 kann ferner eine erste Kommunikationsvorrichtung aufweisen, die derart eingerichtet ist, dass die Erfassungseinheiten 104-1, 104-2, 104-3 mit der Auswertevorrichtung 106 kommunikativ gekoppelt sind (in FIG.1A und FIG.1B mittels der gestrichelten Linie veranschaulicht). Die erste Kommunikationsvorrichtung kann beispielsweise eine Nahfeldkommunikationsvorrichtung (NFC) sein, beispielsweise eine Bluetooth-Kommunikationsvorrichtung. Jede der Erfassungseinheiten 104-1, 104-2, 104-3 kann einen RF Sendeempfänger aufweisen, der zum Senden und Empfangen von HF-Signalen in einem Frequenzbereich in einem Bereich 110 von 100 MHz bis 10 THz, beispielsweise in einem Bereich 110 von 0,1 THz bis 10 THz (beispielsweise entsprechend einer Wellenlänge in einem Bereich von 3 mm bis 30 $\mu$m), eingerichtet ist, beispielsweise in einem Bereich 110 von 1 GHz bis 1 THz

Die Erfassungsvorrichtung 100 kann ferner eine zweite Kommunikationsvorrichtung aufweisen, die derart eingerichtet ist, dass die Auswertevorrichtung 106 mit einer Erfassungsvorrichtung-externen Vorrichtung 304 kommunikativ koppelbar ist. Die zweite Kommunikationsvorrichtung kann beispielsweise eine Weitbereichskommunikationsvorrichtung sein, beispielsweise eine WiFi-, 3G-, 4G-, 5G-Kommunikationsvorrichtung.

Die Erfassungsvorrichtung 100 kann ferner eine Modulationsvorrichtung aufweisen, die eingerichtet ist, die zu sendende erste HF-Strahlung HF zu modulieren, insbesondere mittels FMCW (frequenzmodulierter Dauerstrich)-Modulation oder als eine Pulsfolge.

Die Erfassungsvorrichtung 100 bzw. die Erfassungseinheiten 104 können (jeweils) ferner eine HF-Erzeugungsvorrichtung aufweisen, die eingerichtet ist, die zu sendende erste HF-Strahlung HF zu erzeugen, wobei die HF-Erzeugungsvorrichtung eines aufweist aus einem elektrischen Schwingkreis, einem elektronischen Oszillatorschaltkreis, einem oder mehreren schnellschaltenden Transistoren, einer oder mehreren Resonanztunneldioden oder einem kaskadierten Frequenzvervielfacher.

Als anschauliches Beispiel, die Erfassungseinheit 104 kann zum Senden der HF-Signale eine rein elektronische Struktur bzw. nicht-optische Struktur aufweisen. Die Erfassungseinheit 104 kann zum Senden der HF-Signale auf der Mikrowellen-Frequenzmultiplikationstechnologie basieren. Die Erfassungseinheit 104 kann eine Mikrowellenquelle aufweisen, die einen Gunn-Oszillator und eine Tunneldiode aufweist. Die Mikrowellenquelle kann mit einem Resonator verbunden sein, der ein elektrisches Gleichstromsignal in ein Mikrowellen-Wechselstromsignal umwandelt. Das Mikrowellensignal kann in den Frequenzvervielfacher eingegeben werden, um eine THz-Welle zu erzeugen. Der Frequenzvervielfacher kann eine Schottky-Diode (beispielsweise eine Au-GaAs Schottky-Diode) aufweisen und kaskadiert werden. Dadurch kann eine Mikrowelle mehrfach multipliziert werden, um eine Terahertzwelle (THz-Welle) mit höherer Frequenz zu bilden. Die THz-Welle kann somit vollständig elektronisch in der Erfassungsvorrichtung erzeugt werden. Die elektrische THz-Erzeugung ermöglicht eine kostengünstige und räumlich kompakte HF-Erfassungsvorrichtung 100.

Die HF-Erzeugungsvorrichtung kann beispielsweise einen photoleitenden Emitter aus einem kleinen Stück von Halbleiterkristallen, beispielsweise Galliumarsenid aufweisen. Auf dem Halbleiterkristall können planare Metallelektroden in Form einer Antenne ausgebildet sein. Die Antennen können ein großes elektrisches Feld über ihre Oberfläche aufrechterhalten. Ultraschnelle Lichtimpulse, beispielsweise mit einer Pulslänge von 100 fs, werden auf den Spalt zwischen den Elektroden bei Infrarotwellenlängen fokussiert. Die Photonenenergie der Lichtpulse liegt über der Bandlücke des Halbleiterkristalls. Das erzeugt Elektronen-Loch-Paare, die nahe der Kristalloberfläche angeregt werden, ändern die Leitfähigkeit schnell. Das Anlegen einer Vorspannung beschleunigt das Elektronen-Loch-Paar und führt zu einer schnellen Änderung der Stromdichte. Das Ändern des Dipols erzeugt einen THz-Übergang in der Antenne, der in den freien Raum als THz-Puls abgestrahlt wird. Der Der THz-Puls kann kollimiert und auf den Erfassungsbereich 108 fokussiert werden. Der THz-Puls wird zumindest teilweise von einem Objekt in dem Erfassungsbereich 108 reflektiert. Der reflektierte THz-Puls kann in der Erfassungseinheit 104 rekollimiert werden und auf einen lichtleitenden Empfänger fokussiert werden.

Die Erfassungseinheiten 104 können für den Empfang bzw. die Detektion der zweiten HF-Strahlung basierend auf einer bolometrischen Messung, einem elektrooptischen Abtasten oder einem photoleitenden Empfänger eingerichtet sein.

Die Erfassungsvorrichtung 100 kann ferner eine Energieversorgungsvorrichtung aufweisen, die zum Versorgen der Auswertevorrichtung und der Erfassungseinheiten 104 mit elektrischer Energie eingerichtet ist.

Die Energieversorgungsvorrichtung kann auf oder in dem Träger 102 integriert sein.

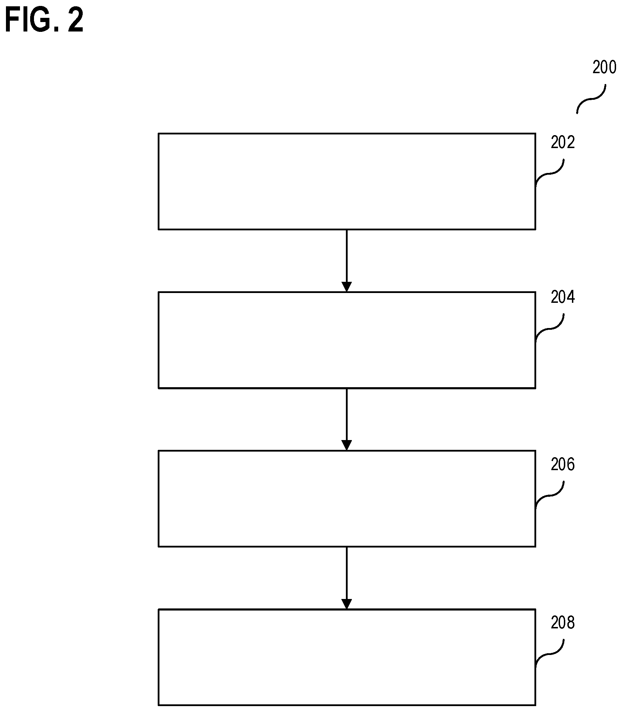

FIG.2 veranschaulicht ein Ablaufdiagram eines Verfahrens 200 zum Betreiben einer Erfassungsvorrichtung gemäß verschiedenen Ausführungsformen. Die Erfassungsvorrichtung 100 kann gemäß einem der beschriebenen Beispiele eingerichtet sein.

Das Verfahren 200 kann ein Ermitteln 202 der Relativposition zwischen der ersten Erfassungseinheit 104-1 und der zweiten Erfassungseinheit 104-2 aufweisen.

Das Verfahren 200 kann ferner ein Emittieren 204 erster HF-Strahlung HF in den Erfassungsbereich 108 mittels der ersten Erfassungseinheit 104-1 und/oder der zweiten Erfassungseinheit 104-2 aufweisen.

Das Verfahren 200 kann ferner ein Erfassen 206 mittels der ersten Erfassungseinheit 104-1 und/oder der zweiten Erfassungseinheit 104-2 zweiter HF-Strahlung HF aufweisen.

Das Verfahren 200 kann ferner ein Bereitstellen 208 mittels der Auswertevorrichtung 106 einer Ausgabe für die erste Erfassungseinheit 104-1 und/oder die zweite Erfassungseinheit 104-2 basierend auf der Relativposition und der/den erfassten zweiten HF-Strahlungen HF aufweisen.

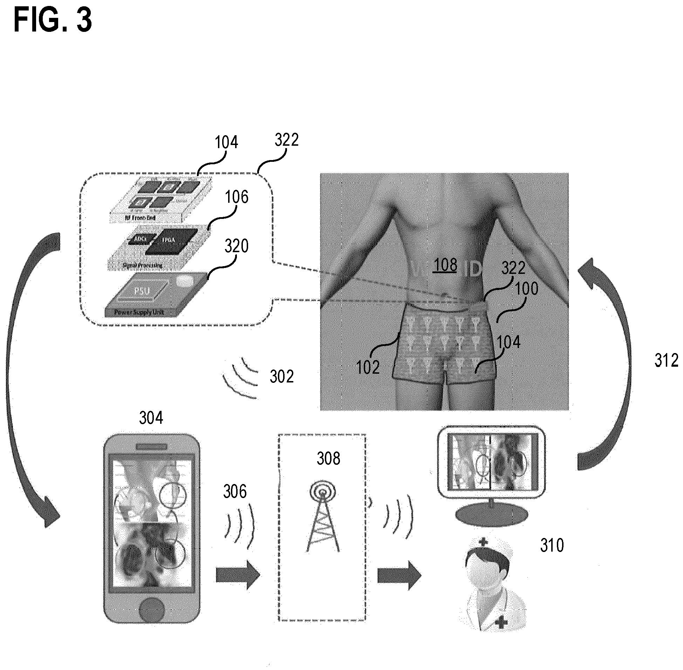

FIG.3 veranschaulicht ein Anwendungsbeispiel einer Erfassungsvorrichtung 100 und eines Verfahrens zum Betreiben einer Erfassungsvorrichtung. Das in FIG.3 veranschaulichte Anwendungsbeispiel ist auf die Untersuchung der Gelenke und Muskulatur im Hüft- bzw. Lendenbereich eines menschlichen Patienten 108 gemäß dem in FIG.1A veranschaulichten Ausführungsbeispiel gerichtet. Der Träger 102 weist in diesem Fall die Form einer Unterhose auf, die von dem Patienten während der Diagnose, beispielsweise während einer Langzeitdiagnose über einen Tag, getragen wird. Auf dem Träger 102, beispielsweise auf dem Gewebe der Unterhose oder in dem Gewebe integriert, befindet sich eine Vielzahl von Antennen einer Vielzahl von Erfassungseinheiten 104.

Auf dem Träger 102, beispielsweise auf dem Gewebe der Unterhose oder in dem Gewebe integriert, kann sich zudem eine Steuereinheit 322 bzw. ein Transceiver 323 befinden. Die Steuereinheit 322 kann einen Teil der Erfassungseinheiten 104 aufweisen, beispielsweise ein oder mehrere RF Front End.

Die Steuereinheit 322 kann alternativ oder zusätzlich die Auswertevorrichtung 106 oder einen Teil davon aufweisen, beispielsweise eine Verstärker- und/oder Filterschaltung.

Die Steuereinheit 322 kann alternativ oder zusätzlich eine Energieversorgungsvorrichtung 320 aufweisen, die zum Versorgen der Auswertevorrichtung und der Erfassungseinheiten 104 mit elektrischer Energie eingerichtet ist.

Die Steuereinheit 322 kann alternativ oder zusätzlich eine erste Kommunikationsvorrichtung 302 aufweisen, beispielsweise eine Bluetooth-Sender oder Sendeempfänger. Mittels der ersten Kommunikationsvorrichtung 302 kann die Ausgabe der Auswertevorrichtung 106 in diesem Anwendungsbeispiel an ein mobiles Kommunikationsendgerät, beispielsweise ein Smartphone, des Patienten übermittelt werden.

Die Erfassungsvorrichtung 100 kann ferner eine zweite Kommunikationsvorrichtung 306 (in dem Anwendungsbeispiel eine Kombination aus Smartphone des Patienten und der entsprechenden Anwendung auf dem Smartphone) aufweisen, um die Ausgabe der Auswertevorrichtung ggfs. aufbereitet oder weiterverarbeitet mittels eines Weitbereichsnetzes 308, beispielsweise Internet, 4G, 5G; an entferntes medizinisches Personal 310 zu übermitteln. Das medizinische Personal 310 kann anhand der übermittelten Daten eine Ferndiagnose 312 erstellen und einen entsprechenden individualisierten Behandlungsplan erstellen.

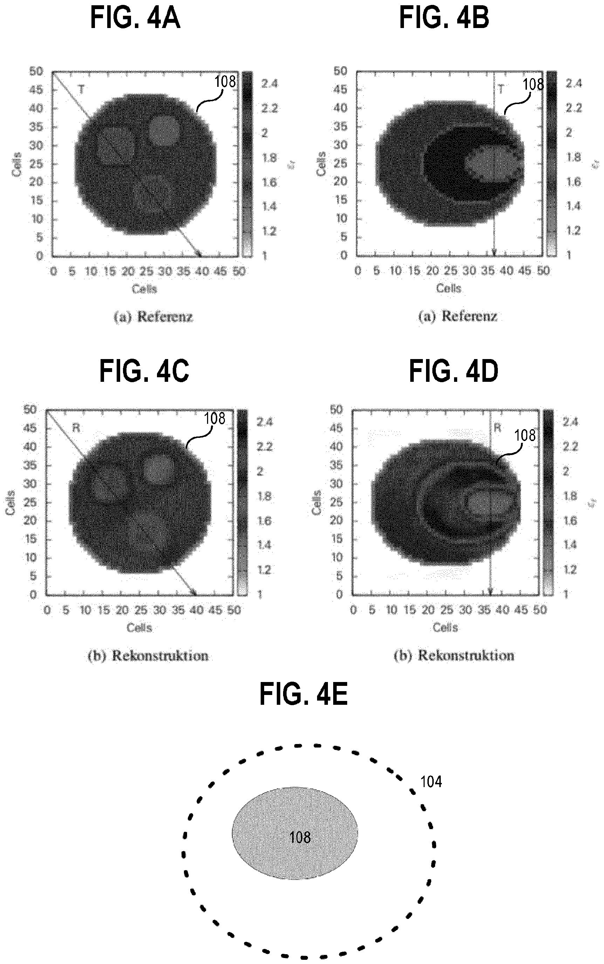

FIG.4A-E veranschaulichen ein Anwendungsbeispiel einer Erfassungsvorrichtung und eines Verfahrens zum Betreiben einer Erfassungsvorrichtung. FIG.4B veranschaulicht eine Schnittansicht eines in FIG.4E veranschaulichten Referenzobjektes, das mittels einer zuvor beschriebenen Erfassungsvorrichtung 100 untersucht wurde. FIG.4A ist eine andere Schnittansicht des Referenzobjektes der FIG.4E. FIG.4C und FIG.4D veranschaulichen zu FIG.4A bzw. FIG.4B korrespondierende Rekonstruktionen der Schnittansichten, die sich aus den Messungen mittels der Erfassungsvorrichtung 100 ergeben. Es ist ersichtlich, dass die interne Struktur des Referenzobjektes korrekt wiedergeben werden kann mittels der Erfassungsvorrichtung 100.

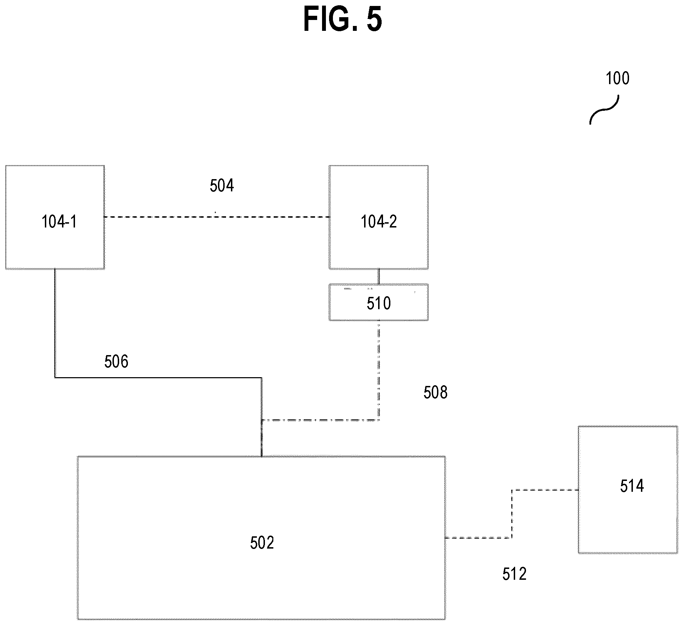

FIG.5 veranschaulicht ein Diagramm einer Erfassungsvorrichtung.

Die Erfassungsvorrichtung 100 kann eine zentrale Auswerte- und Prozessierungseinheit 502 aufweisen. Die Auswerte- und Prozessierungseinheit 502 kann eingerichtet sein, die Energieversorgung, Signalerzeugung, Signalverstärkung, Filterung und/oder Digitalisierung der empfangenen (zweiten) HF-Signale von allen Erfassungseinheiten 104-1, 104-2, beispielsweise Antennen 104-1, 104-2, zu übernehmen bzw. zu hosten.

Die Erfassungseinheiten können untereinander kommunikativ verbunden sein 504, beispielsweise zur Funk Puls Synchronisation oder zur Phasensynchronisation.

Die Erfassungseinheiten 104-1, 104-2 können elektrisch, beispielsweise mittels miniaturisierter Coax-Kabel (auch als Koaxialkabel bezeichnet) 506, oder photonisch durch Lichtwellenleiter 518 mit der Auswerte- und Prozessierungseinheit 502 gekoppelt sein. Bei einer photonischen Kopplung kann ein Wandler 510 vorgesehen sein, der auf/an den Erfassungseinheiten 104-1, 104-2 eine Wandlung vom photonisch geführten Signalen in elektrische Signale durchführt.

Ein Lichtwellenleiter 1508 und/oder optischer Wandler 510 kann räumlich sehr dünn bzw. flach ausgebildet sein und in Textilgewebe eines textilen Trägers eingewebt werden. Alternativ kann jedoch auch eine Verbindung 506 mittels eines Koaxialkabels oder drahtlos erfolgen.

Die Auswerte- und Prozessierungseinheit 502 kann mittels einer Funkverbindung 512, beispielsweise eine drahtlose Bereichsnetzwerkverbindung (WLAN) mit einer Anzeige- und Verarbeitungseinheit 514, beispielsweise einem Smartphone, verbunden sein.

Alternativ oder zusätzlich kann jede Erfassungseinheit 104 der Erfassungsvorrichtung 100 eine Auswerte- und Prozessierungseinheit aufweisen. Die Auswerte- und Prozessierungseinheit der Erfassungseinheit(en) 104 kann (jeweils) eingerichtet sein, die Energieversorgung, Signalerzeugung, beispielsweise frequenzmodulierte Dauerstrich-Modulation (FMCW) oder Puls-Modulation; Signalverstärkung, Filterung, Digitalisierung des empfangenen, zweiten HF-Signals Empfangssignals der Erfassungseinheit 104 zu übernehmen bzw. zu hosten.

An jeder Erfassungseinheit 104 kann dazu ein digitales Basisband gegeben sein. Die einzelnen Erfassungseinheiten 104 können über ein sogenanntes Mesh-Netzwerk 504, beispielsweise WLAN, untereinander verbunden sein. Über diese Verbindung 504 kann die Konfiguration, beispielsweis Synchronisation, beispielsweise Phasensynchronisation, der einzelnen Erfassungseinheiten 104 erfolgen; die Koordination der Messungen erfolgen, beispielsweise welche Erfassungseinheit 104 sendet ein erstes HF-Signal zu welcher Zeit bzw. welchem Zeitpunkt und welche Erfassungseinheit(en) 104 sollen dieses Signal als zweites HF-Signal erfassen. Alternativ oder zusätzlich kann der Austausch der Messdaten und/oder die Übertragung der Messdaten zu einer externen Anzeige/Verarbeitungseinheit 514 von der jeweiligen Auswerte- und Prozessierungseinheit gesteuert werden. Die Synchronisation der Signale der Erfassungseinheiten 104 kann beispielsweise über ein Pilotsignal in einem anderen Frequenzbereich als dem Frequenzbereich des ersten HF-Signals und des zweiten HF-Signals erfolgen.

Alternativ können für die Synchronisation der Frequenz oder Phase der Signale der Erfassungseinheiten 104 und/oder zum Übertragen von Messwerten auch kabelgebundene Verbindungen 504, beispielsweise Coaxkabel, zwischen den Erfassungseinheiten 104 verwendet werden.

Die Erfassungseinheit(en) 140 mit dem kombinierten Antennen-Prozessierungselement können beispielsweise miniaturisiert als dedizierte Chips implementiert sein. Der Chip kann mit einem Antennenelement, beispielsweise durch Bonding, integriert oder verbunden auf dem flexiblen Träger, beispielsweise einem Textilstoff, angebracht oder eingewoben sein.

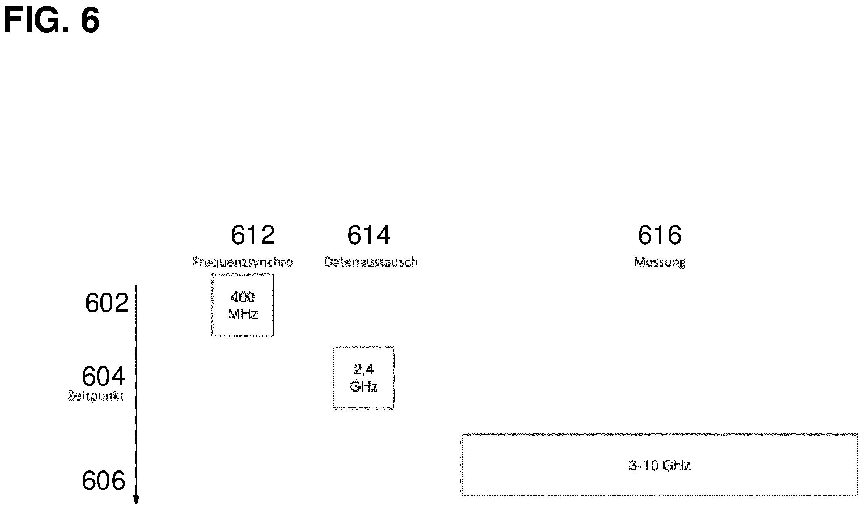

FIG.6 veranschaulicht ein Timing-Diagramm eines Verfahrens zum Betreiben einer Erfassungsvorrichtung unter Bezugnahme der in FIG.5 veranschaulichten Erfassungsstruktur.

Zu einem ersten Zeitpunkt 602 kann eine Pilot-Ton 612 an die Erfassungseinheit(en) 140 ausgegeben werden, beispielsweise mittels eines Funksignals in einem Frequenzband bei 400 MHz. Der Pilot-Ton kann anschaulich ein Trigger-Signal oder Wake-up Signal sein. Mittels des Pilot-Tons können die Erfassungseinheit(en) 140 aus einem Stand by-Zustand oder Sleep-Zustand in einen aktiven Betriebszustand versetzt werden.

Zwischen den Erfassungseinheit(en) 140 kann zu einem zweiten Zeitpunkt 604, beispielsweise im aktiven Betriebszustand, über die Verbindung 504 ein Datenaustausch 614 erfolgen. Die Verbindung 504 kann beispielsweise in einem Frequenzband bei 2,4 GHz erfolgen. Der Datenaustausch 614 kann beispielweise ein Messprotokoll aufweisen. Ein Messprotokoll kann beispielsweise eine Kalibrierung der räumlichen Relativpositionen der Erfassungseinheit(en) 140 aufweisen. Ein Messprotokoll kann jedoch auch die Rollenverteilung der Erfassungseinheit(en) 140 aufweisen, beispielsweise welche der Erfassungseinheit(en) 140 das erste HF-Signal sendet und welche der Erfassungseinheit(en) 140 das zweite HF-Signal, das zuvor von der sendenden Erfassungseinheit 140 emittiert wurde, empfängt. Das Messprotokoll kann weitere Angaben aufweisen, beispielsweise die Frequenz und/oder Phase des ersten HF-Signals und/oder der Messzeitpunkt 606 der Messung 616. Die Messung kann beispielsweise in einem Frequenzbang von 3 GHz bis 10 GHz erfolgen.

Die Signale 612, 614, 616 können somit in unterschiedlichen Frequenzbändern und/oder unterschiedlichen Zeitpunkten 602, 604, 606 übermittelt werden und können sich somit nicht stören.

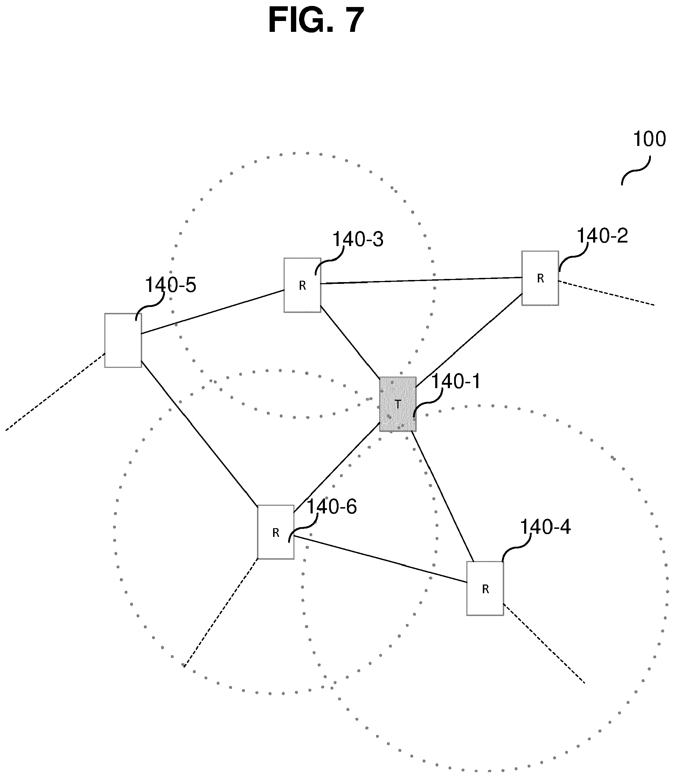

FIG.7 veranschaulicht ein Diagramm eines Verfahrens zum Betreiben einer Erfassungsvorrichtung, beispielsweise zur Kalibration der Erfassungseinheiten 140, beispielsweise der Relativpositionen der Erfassungseinheiten 140 zueinander.

Für die Kalibration und Positionsermittlung der Erfassungseinheiten 140 (in FIG.7 insbesondere 140-1, 1402, 140-3, 140-4, 140-5, 140-6) sind die Nachbarschaftsbeziehungen der Erfassungseinheiten 140 bekannt.

Die Positionskalibration der Erfassungseinheiten 140 kann mittels Trilateration des äußeren Pfades zwischen benachbarten Erfassungseinheiten 140, beispielsweise zwischen einer Erfassungseinheit 140-1 relativ zu den direkt benachbarten Erfassungseinheiten 140,3, 1406, und 140-4 (in FIG. 7 mittels der gepunkteten Linien veranschaulicht).

Der äußere Pfad ist der Pfad zwischen dem Träger der Erfassungsvorrichtung und dem zu untersuchenden Körper (auf der Körperoberfläche) bzw. dem zu untersuchenden Gewebe. Das erste Signal läuft anschaulich als Oberflächenwellen direkt (ohne in das zu untersuchende Gewebe einzudringen) von der sendenden ersten Erfassungsvorrichtung 140-1 zu den empfangenden (zweiten) Erfassungsvorrichtungen 140-3, 140-4, 140-6. Es kann somit direkt das Messsignal, das keine Informationen des zu untersuchenden Gewebes enthält, für die Kalibrierung verwendet (auch als Kalibrationssignal bezeichnet). Das Kalibrationssignal breitet sich im zumindest teilweisen Freiraum zwischen benachbarten Erfassungsvorrichtung 140-1 zu 140-3, 140-4, 140-6 entlang des äußeren Pfades aus und weist von den empfangenen zweiten HF-Signalen die stärkste Signalkomponente auf. Das Kalibrationssignal wird zudem zeitlich vor den Signalen mit den Informationen über das zu untersuchende Gewebe empfangen. Somit ist das Kalibrationssignal in den zweiten HF-Signalen an der Signalstärke und dem Erfassungszeitpunkt ermittelbar. Mittels Trilateration, beispielsweise aus der Laufzeitmessung des Kalibrationssignal zwischen sendender Erfassungseinheit 140-1 und empfangender Erfassungseinheit 140-3, 140-4, 140-6 kann der Abstand und Position der einzelnen Erfassungseinheiten 140-1, 140-3, 140-4, 140-6 ermittelt werden.

Eine Kalibrationsmessung kann anwendungsspezifisch für alle Erfassungseinheiten 140 in unterschiedlicher Rollenverteilung (Sender/Empfänger) durchgeführt werden.

Nach einer erfolgten initialen Kalibration kann der Phasenunterschied zwischen aufeinander folgenden Messungen der entsprechenden Sender-Empfänger-Kombinationen der Erfassungseinheiten 140 für die Trilateration verwendet werden. Der Phasenunterschied kann in einen Laufzeitunterschied umgerechnet werden und zu den ursprünglichen Laufzeiten aus der initialen (vollständigen) Kalibrationsmessung addiert werden.

Eine sendende Erfassungseinheiten 140-1 kann an mehrere empfangende Erfassungseinheiten 140-3, 140-4, 140-6 gleichzeitig das erste HF-Signal senden. Dies ermöglicht eine schnellere Kalibration.

Zur Trilateration kann die Grundkonfiguration der Erfassungsvorrichtung berücksichtigen werden, beispielsweise die Anordnung mit den Nachbarschaftsbeziehungen und Abständen der Erfassungseinheiten 140 auf dem Träger der Erfassungsvorrichtung 100.

Für jede Sender-Empfänger-Kombination des Systems kann eine Messung wie folgt ablaufen:

Frequenz- und Zeitsynchronisation von sendender Erfassungseinheit 140-1 und empfangenden Erfassungseinheiten 140-3, 140-4, 140-6 (beispielsweise 3 oder mehr Erfassungseinheiten 140), beispielsweise mittels eines Pilot-Tons oder Pilot-Signals (siehe oben) über eine digitale Verbindung bzw. Schnittstelle, beispielsweise Verbindung 504, 506, 510 (siehe FIG.5),

Senden des ersten HF-Signals durch die erste Erfassungseinheit 140-1,

Empfangen des zweiten HF-Signals, welches das Kalibrationssignal aufweist, durch die zweiten Erfassungseinheiten 140-3, 140-4, 140-6,

Ermitteln der Laufzeit des gesendeten HF-Signals von der ersten Erfassungseinheit 140-1 zu jeder der weiteren HF-Erfassungeinheit 140-3, 140-4, 140-6 als Differenz zwischen Sendezeitpunkt des ersten HF-Signals zum Empfangszeitpunkt des zweiten HF-Signals unter Verwendung des Kalibrationssignals, und

Ermitteln des räumlichen Abstandes zwischen der ersten Erfassungseinheit 140-1 und jeder der zweiten Erfassungseinheiten 140-3, 140-4, 140-6.

Die Ermittlung des Abstandes kann auf der Annahme einer Freiraumausbreitung basieren, wobei die Freiraumgeschwindigkeit die Geschwindigkeit des gesendeten HF-Signals entlang der Oberfläche des zu untersuchenden Körpers (des Kalibrationssignals) ist.

Bei den Messungen 616 (siehe FIG.6) kann die Sender/Empfänger-Rollenverteilung der Erfassungseinheiten anwendungsspezifisch durchgeschaltet bzw. permutiert werden. Die Nachbarschaftsbeziehung der Erfassungseinheiten ist bei den Messungen 616 die gleiche wie bei der Kalibrierung und bekannt.

Aus allen Abstandsmessungen der einzelnen Sender-Empfängerpaare der Erfassungseinheiten 140 kann mittels Trilateration die Positionen der Erfassungseinheiten 140 ermittelt werden.

Eine Kalibration der Positionen der Erfassungseinheiten 140 kann anwendungsspezifisch erfolgen, beispielsweise bei jeder Messung oder in einem vorbestimmten Intervall, beispielsweise nach einer vorgegebenen Zeit oder nach einer vorgegebenen Anzahl an Messungen.

Nach einer initialen Kalibrierung kann bei den Messungen 616 (siehe FIG.6) eine Frequenz ausgewählt werden, um die Informationen des Erfassungsbereiches zu ermitteln. Die Phasenunterschiede der von den Erfassungseinheiten erfassten (zweiten) Signale korrespondieren zu Laufzeitunterschieden der Signal. Dadurch kann relativ zum initialen Zustand, beispielsweise der räumlichen Anordnung der Erfassungseinheiten 140 zueinander, eine Positionsänderung der Erfassungseinheiten zueinander ermittelt werden. Gegebenenfalls können die Positionsinformationen der Erfassungseinheiten für die Auswertung der Messignale 616 nachkalibriert werden.

Im Folgenden werden verschiedene Beispiele beschrieben, die sich auf das hierin Beschriebene und in den Figuren Dargestellte beziehen.

Beispiel 1 ist eine Erfassungsvorrichtung, aufweisend einen verformbaren Träger, derart eingerichtet, dass ein vorbestimmter Bereich zumindest teilweise von dem Träger umgebbar ist; mindestens eine erste Erfassungseinheit und eine zweite Erfassungseinheit, die an dem Träger befestigt sind und jeweils eingerichtet sind, HF-Strahlung in Richtung eines Erfassungsbereiches zu emittieren und HF-Strahlung aus dem Erfassungsbereich zu empfangen, wobei die empfangene HF-Strahlung eine oder mehrere Eigenschaften des Erfassungsbereichs repräsentiert und auf der emittieren HF-Strahlung basiert; wobei der Träger derart verformbar eingerichtet ist, dass eine Relativposition der ersten Erfassungseinheit zu der zweiten Erfassungseinheit aufgrund einer Verformung des Trägers veränderbar ist; und eine Auswertevorrichtung, die eingerichtet ist, die Relativposition der ersten und zweiten Erfassungseinheit zu ermitteln und eingerichtet ist, eine Ausgabe bereitzustellen basierend auf der ermittelten Relativposition der ersten und zweiten Erfassungseinheit und der mittels der ersten Erfassungseinheit und der zweiten Erfassungseinheit empfangenen HF-Strahlung.

In Beispiel 2 weist das Beispiel 1 optional auf, dass der vorbestimmte Bereich zumindest teilweise den Erfassungsbereich umfasst. Alternativ oder zusätzlich ist der vorbestimmte Bereich zumindest teilweise durch den Träger von dem Erfassungsbereich separiert.

In Beispiel 3 weist das Beispiel 1 oder 2 optional auf, dass die Auswertevorrichtung eingerichtet ist, die Relativposition der ersten und zweiten Erfassungseinheit vor der Emission der ersten HF-Strahlung zu ermitteln. Alternativ oder zusätzlich ist die Auswertevorrichtung eingerichtet, die Relativposition der ersten und zweiten Erfassungseinheit basierend auf der empfangenen zweiten Strahlung zu ermitteln.

In Beispiel 4 weist eines der Beispiele 1 bis 3 optional auf, dass die erste Erfassungseinheit und die zweite Erfassungseinheit jeweils mindestens einen Radarsensor aufweist.

In Beispiel 5 weist eines der Beispiele 1 bis 4 optional auf, dass der Erfassungsbereich einen zeitlich veränderlichen Raum umfasst, wobei die zeitliche Veränderung mindestens eines umfasst aus dem Volumen, der Zusammensetzung und/oder der relativen Ausrichtung.

In Beispiel 6 weist eines der Beispiele 1 bis 5 optional ferner mindestens eine dritte Erfassungseinheit auf, die an dem Träger befestigt ist und eingerichtet ist, eine erste HF-Strahlung in Richtung des Erfassungsbereiches zu emittieren und eine zweite HF-Strahlung aus dem Erfassungsbereich zu empfangen, wobei die zweite HF-Strahlung eine oder mehrere Eigenschaften des Erfassungsbereichs repräsentiert und auf der ersten HF-Strahlung basiert.

In Beispiel 7 weist eines der Beispiele 1 bis 6 optional auf, dass der Träger eine Befestigungsstruktur aufweist, die derart eingerichtet ist, dass sich der Träger verformt, wenn sich der Erfassungsbereich verformt.

In Beispiel 8 weist eines der Beispiele 1 bis 4 optional auf, dass die Auswertevorrichtung zumindest teilweise auf dem Träger integriert ist.

In Beispiel 9 weist eines der Beispiele 1 bis 8 optional ferner eine Kommunikationsvorrichtung auf, die derart eingerichtet ist, dass die Erfassungseinheiten mit der Auswertevorrichtung kommunikativ gekoppelt sind.

In Beispiel 10 weist eines der Beispiele 1 bis 9 optional ferner eine Kommunikationsvorrichtung auf, die derart eingerichtet ist, dass die Auswertevorrichtung mit einer Erfassungsvorrichtung-externen Vorrichtung (304) kommunikativ koppelbar ist.

In Beispiel 11 weist eines der Beispiele 1 bis 10 optional auf, dass jede der Erfassungseinheiten einen RF Sendeempfänger aufweist, der um Senden und Empfangen von HF-Signalen in einem Frequenzbereich in einem Bereich von 100 MHz bis 10 THz, aufweist, insbesondere in einem Bereich von 1 GHz bis 1 THz.

In Beispiel 12 weist eines der Beispiele 1 bis 11 optional auf, dass jede der Erfassungseinheiten mindestens eine Antenne aufweist, die zum Emittieren der ersten HF-Strahlung und/oder Empfangen der zweiten HF-Strahlung eingerichtet ist.

In Beispiel 13 weist eines der Beispiele 1 bis 12 optional auf, dass jede der Erfassungseinheiten ein Antennen-Array mit einer Vielzahl von Antennen aufweist, die jeweils oder zusammen zum Emittieren der ersten HF-Strahlung und/oder Empfangen der zweiten HF-Strahlung eingerichtet sind.

In Beispiel 14 weist eines der Beispiele 1 bis 13 optional auf, dass die Auswertevorrichtung eine Radar-Bildgebung umfasst.

In Beispiel 15 weist eines der Beispiele 1 bis 14 ferner eine Anzeigevorrichtung aufweisen, die eingerichtet ist, die Ausgabe anzuzeigen.

In Beispiel 16 weist eines der Beispiele 1 bis 15 ferner eine Modulationsvorrichtung auf, die eingerichtet ist, die zu sendende erste HF-Strahlung zu modulieren, insbesondere mittels FMCW-Modulation oder als eine Pulsfolge.

In Beispiel 17 weist eines der Beispiele 1 bis 16 optional ferner eine HF-Erzeugungsvorrichtung auf, die eingerichtet ist, die zu sendende HF-Strahlung zu erzeugen, wobei die HF-Erzeugungsvorrichtung eines aufweist aus einem elektrischen Schwingkreis, einem elektronischen Oszillatorschaltkreis, einem oder mehreren schnellschaltenden Transistor(en), einer oder mehreren Resonanztunneldiode(n) oder einem kaskadierten Frequenzvervielfacher.

In Beispiel 18 weist eines der Beispiele 1 bis 17 optional auf, dass die erste HF-Strahlung und die zweite HF-Strahlung die gleiche Frequenz aufweisen.

Beispiel 19 ist ein Verfahren zum Betreiben einer Erfassungsvorrichtung. Die Erfassungsvorrichtung kann gemäß einem der Beispiele 1 bis 18 eingerichtet sein. Das Verfahren kann aufweisen: Ermitteln der Relativposition zwischen der ersten Erfassungseinheit und der zweiten Erfassungseinheit; Emittieren erster HF-Strahlung in den Erfassungsbereich mittels der ersten Erfassungseinheit und/oder der zweiten Erfassungseinheit; Erfassen mittels der ersten Erfassungseinheit und/oder der zweiten Erfassungseinheit zweiter HF-Strahlung; Bereitstellen mittels der Auswertevorrichtung einer Ausgabe für die erste Erfassungseinheit und/oder die zweite Erfassungseinheit basierend auf der Relativposition und der/den erfassten zweiten HF-Strahlung(en).

In Beispiel 20 kann der Gegenstand von Beispiel 19 optional aufweisen, dass der vorbestimmte Bereich zumindest teilweise den Erfassungsbereich umfasst, oder der vorbestimmte Bereich zumindest teilweise durch den Träger von dem Erfassungsbereich separiert ist.

Es versteht sich, dass Funktionen, Algorithmen, etc. die hierin mit Bezug auf ein Verfahren beschrieben sind auch in gleicher oder ähnlicher Weise in einer Vorrichtung implementiert sein können und umgekehrt.

## Patentansprüche

1. Erfassungsvorrichtung (100), aufweisend:

    einen verformbaren Träger (102), derart eingerichtet, dass ein vorbestimmter Bereich (110) zumindest teilweise von dem Träger (102) umgebbar ist,

    mindestens eine erste Erfassungseinheit (104-1) und eine zweite Erfassungseinheit (104-2), die an dem Träger (102) befestigt sind und jeweils eingerichtet sind, erste HF-Strahlung (HF) in Richtung eines Erfassungsbereiches (108) zu emittieren und zweite HF-Strahlung (HF) aus dem Erfassungsbereich (108) zu empfangen, wobei die empfangene zweite HF-Strahlung (HF) eine oder mehrere Eigenschaften des Erfassungsbereichs (108) repräsentiert und auf der emittierten ersten HF-Strahlung (HF) basiert;
    wobei der Träger (102) derart verformbar eingerichtet ist, dass eine Relativposition der ersten Erfassungseinheit (104-1) zu der zweiten Erfassungseinheit (104-2) aufgrund einer Verformung des Trägers (102) veränderbar ist; und

    eine Auswertevorrichtung (106), die eingerichtet ist, die Relativposition der ersten und zweiten Erfassungseinheit (104-1, 104-2) zu ermitteln und eingerichtet ist, eine Ausgabe bereitzustellen basierend auf der ermittelten Relativposition der ersten und zweiten Erfassungseinheit (104-1, 104-2) und der mittels der ersten Erfassungseinheit (104-1) und der zweiten Erfassungseinheit (104-2) empfangenen zweiten HF-Strahlung (HF); und
    wobei die Auswertevorrichtung (106) eingerichtet ist, die Relativposition der ersten und zweiten Erfassungseinheit (104-1, 104-2) basierend auf der empfangenen zweiten HF-Strahlung zu ermitteln.

2. Erfassungsvorrichtung (100) gemäß Anspruch 1,
   wobei der vorbestimmte Bereich (110) zumindest teilweise durch den Träger (102) von dem Erfassungsbereich (108) separiert ist.

3. Erfassungsvorrichtung (100) gemäß Anspruch 1,
   wobei der vorbestimmte Bereich (110) zumindest teilweise den Erfassungsbereich (108) umfasst.

4. Erfassungsvorrichtung (100) einem der Ansprüche 1 bis 3,
   wobei die erste Erfassungseinheit (104-1) und die zweite Erfassungseinheit (104-2) jeweils mindestens einen Radarsensor aufweist.

5. Erfassungsvorrichtung (100) gemäß einem der Ansprüche 1 bis 4,
   wobei der Erfassungsbereich (108) einen zeitlich veränderlichen Raum umfasst, wobei die zeitliche Veränderung mindestens eine Veränderung des Volumens, eine Veränderung der Zusammensetzung und/oder eine Veränderung der relativen Ausrichtung aufweist.

6. Erfassungsvorrichtung (100) gemäß einem der Ansprüche 1 bis 5,
   ferner aufweisend eine Kommunikationsvorrichtung, die derart eingerichtet ist, dass die Auswertevorrichtung (106)

mit einer Erfassungsvorrichtung-externen Vorrichtung (304) kommunikativ koppelbar ist.

7.  Erfassungsvorrichtung (100) gemäß einem der Ansprüche 1 bis 6,
    wobei jede der Erfassungseinheiten (104-1, 104-2, 104-3) einen RF-Sendeempfänger aufweist, der zum Senden und Empfangen von elektromagnetischen HF-Signalen in einem Frequenzbereich in einem Bereich (110) von 100 MHz bis 10 THz, eingerichtet ist, insbesondere in einem Bereich (110) von 1 GHz bis 1 THz.

8.  Erfassungsvorrichtung (100) gemäß einem der Ansprüche 1 bis 7,
    wobei jede der Erfassungseinheiten (104-1, 104-2, 104-3) mindestens eine Antenne aufweist, die zum Emittieren der ersten HF-Strahlung (HF) und/oder Empfangen der zweiten HF-Strahlung (HF) eingerichtet ist.

9.  Erfassungsvorrichtung (100) gemäß einem der Ansprüche 1 bis 8,
    wobei jede der Erfassungseinheiten (104-1, 104-2, 104-3) ein Antennen-Array mit einer Vielzahl von Antennen aufweist, die jeweils oder zusammen zum Emittieren der ersten HF-Strahlung (HF) und/oder Empfangen der zweiten HF-Strahlung (HF) eingerichtet sind.

10. Erfassungsvorrichtung (100) gemäß einem der Ansprüche 1 bis 13,
    wobei die Auswertevorrichtung (106) eine Radar-Bildgebung umfasst.

11. Erfassungsvorrichtung (100) gemäß einem der Ansprüche 1 bis 10, ferner aufweisend eine Anzeigevorrichtung, die eingerichtet ist, die Ausgabe anzuzeigen.

12. Erfassungsvorrichtung (100) gemäß einem der Ansprüche 1 bis 11, ferner aufweisend eine Modulationsvorrichtung, die eingerichtet ist, die zu sendende erste HF-Strahlung (HF) zu modulieren, insbesondere mittels FMCW-Modulation oder als eine Pulsfolge.

13. Erfassungsvorrichtung (100) gemäß einem der Ansprüche 1 bis 12, ferner aufweisend eine HF-Erzeugungsvorrichtung eingerichtet, die zu sendende HF-Strahlung (HF) zu erzeugen, wobei die HF-Erzeugungsvorrichtung eines aufweist aus einem elektrischen Schwingkreis, einem elektronischen Oszillatorschaltkreis, einem oder mehreren schnellschaltenden Transistor(en), einer oder mehreren Resonanztunneldiode(n) oder einem kaskadierten Frequenzvervielfacher.

14. Erfassungsvorrichtung (100) gemäß einem der Ansprüche 1 bis 13, wobei die erste HF-Strahlung (HF) und die zweite HF-Strahlung (HF) die gleiche Frequenz aufweisen.

15. Verfahren zum Betreiben einer Erfassungsvorrichtung (100), die Erfassungsvorrichtung (100) aufweisend:

    einen verformbaren Träger (102), derart eingerichtet, dass ein vorbestimmter Bereich (110) zumindest teilweise von dem Träger (102) umgebbar ist;

    mindestens eine erste Erfassungseinheit (104-1) und eine zweite Erfassungseinheit (104-2), die an dem Träger (102) befestigt sind und jeweils eingerichtet sind, eine erste HF-Strahlung (HF) in Richtung eines Erfassungsbereiches (108) zu emittieren und eine zweite HF-Strahlung (HF) aus dem Erfassungsbereich (108) zu empfangen, wobei die empfangene zweite HF-Strahlung (HF) eine oder mehrere Eigenschaften des Erfassungsbereichs (108) repräsentiert und auf der emittierten ersten HF-Strahlung (HF) basiert; wobei der Träger (102) derart verformbar eingerichtet ist, dass eine Relativposition der ersten Erfassungseinheit (104-1) zu der zweiten Erfassungseinheit (104-2) aufgrund einer Verformung des Trägers (102) veränderbar ist; und

    eine Auswertevorrichtung (106), die eingerichtet ist, die Relativposition der ersten und zweiten Erfassungseinheit (104-1, 104-2) zu ermitteln und eingerichtet ist, eine Ausgabe bereitzustellen basierend auf der ermittelten Relativposition der ersten und zweiten Erfassungseinheit (104-1, 104-2) und der mittels der ersten Erfassungseinheit (104-1) und der zweiten Erfassungseinheit (104-2) empfangenen HF-Strahlung (HF), wobei die Auswertevorrichtung (106) eingerichtet ist, die Relativposition der ersten und zweiten Erfassungseinheit (104-1, 104-2) basierend auf der empfangenen zweiten HF-Strahlung zu ermitteln;

    das Verfahren aufweisend:

Ermitteln der Relativposition zwischen der ersten Erfassungseinheit (104-1) und der zweiten Erfassungseinheit (104-2);

Emittieren einer ersten HF-Strahlung (HF) in den Erfassungsbereich (108) mittels der ersten Erfassungseinheit (104-1) und/oder der zweiten Erfassungseinheit (104-2);

Erfassen mittels der ersten Erfassungseinheit (104-1) und/oder der zweiten Erfassungseinheit (104-2) einer zweiten HF-Strahlung (HF);

Bereitstellen mittels der Auswertevorrichtung (106) einer Ausgabe für die erste Erfassungseinheit (104-1) und/oder die zweite Erfassungseinheit (104-2) basierend auf der Relativposition und der erfassten zweiten HF-Strahlung (HF).

# FIG. 1A

# FIG. 1B

# FIG. 2

200

202

204

206

208

# FIG. 3

## FIG. 4A

(a) Referenz

## FIG. 4B

(a) Referenz

## FIG. 4C

(b) Rekonstruktion

## FIG. 4D

(b) Rekonstruktion

## FIG. 4E

# FIG. 5

100

104-1

504

104-2

510

506

508

502

512

514

# FIG. 6

612
Frequenzsynchro

614
Datenaustausch

616
Messung

602

604
Zeitpunkt

606

400
MHz

2,4
GHz

3-10 GHz

# FIG. 7

Europäisches
Patentamt

European
Patent Office

Office européen
des brevets

## EUROPÄISCHER RECHERCHENBERICHT

Nummer der Anmeldung

EP 21 19 4550

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2015/208949 A1 (TUPIN JR JOE P [US] ET AL) 30. Juli 2015 (2015-07-30) <br> * Abbildungen 4,5,12,33; Tabelle 2 * <br> * Absatz [0145] – Absatz [0147] * <br> * Absatz [0156] – Absatz [0161] * <br> * Absatz [0176] – Absatz [0179] * <br> * Absatz [0219] * <br> ----- | 1-15 | INV. <br> A61B5/0507 <br> A61B5/00 <br> A61B5/11 <br> G01S13/00 |
| X | US 9 924 921 B1 (IRISH LINDA [US] ET AL) 27. März 2018 (2018-03-27) <br> * Abbildungen 6,7 * <br> * Spalte 14, Zeile 32 – Spalte 15, Zeile 14 * <br> ----- | 1,15 | |

RECHERCHIERTE SACHGEBIETE (IPC)

A61B
G01S

Der vorliegende Recherchenbericht wurde für alle Patentansprüche erstellt

| Recherchenort | Abschlußdatum der Recherche | Prüfer |
|---|---|---|
| Den Haag | 21. Januar 2022 | Oancea, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 21 19 4550

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-01-2022

| Im Recherchenbericht angeführtes Patentdokument | Datum der Veröffentlichung | Mitglied(er) der Patentfamilie | Datum der Veröffentlichung |
|---|---|---|---|
| US 2015208949 A1 | 30-07-2015 | US 2011060215 A1 | 10-03-2011 |
| | | US 2015208949 A1 | 30-07-2015 |
| | | US 2017258366 A1 | 14-09-2017 |
| US 9924921 B1 | 27-03-2018 | KEINE | |

Für nähere Einzelheiten zu diesem Anhang : siehe Amtsblatt des Europäischen Patentamts, Nr.12/82